# EUROPÄISCHE PATENTSCHRIFT

(11) **EP 1 127 050 B1**
(45) Veröffentlichungstag und Bekanntmachung des Hinweises auf die Patenterteilung: **25.08.2010**
(21) Anmeldenummer: 99955919.8
(22) Anmeldetag: 30.10.1999
(51) Int. Cl.: C07D 231/18, C07C 243/30, C07C 241/04

(54) **VERFAHREN ZUR HERSTELLUNG VON 1-SUBSTITUIERTEN 5-HYDROXYPYRAZOLEN**
METHOD FOR PRODUCING 1-SUBSTITUTED 5-HYDROXYPYRAZOLES
PROCEDE DE FABRICATION DE 5-HYDROXYPYRAZOLES 1-SUBSTITUES

(30) Priorität: 05.11.1998 DE 19851100; 10.03.1999 DE 19910505
(43) Veröffentlichungstag der Anmeldung: 29.08.2001
(73) Patentinhaber: BASF SE, 67056 Ludwigshafen (DE)
(72) Erfinder: GÖTZ, Norbert, D-67547 Worms (DE); GÖTZ, Roland, D-68809 Neulu heim (DE); RACK, Michael, D-69123 Heidelberg (DE)
(74) Vertreter: Reitstötter - Kinzebach
(86) Internationale Anmeldenummer: PCT/EP1999/008275
(87) Internationale Veröffentlichungsnummer: WO 2000/027821

(56) Entgegenhaltungen:
- EP-A- 0 203 428
- EP-A- 0 240 001
- CHEMICAL ABSTRACTS, vol. 115, no. 5, 5. August 1991 (1991-08-05) Columbus, Ohio, US; abstract no. 049683g, TSUNODA T ET AL: "Preparation of 5-hydroxypyrazole derivatives" Seite 849; XP002130078 -& JP 03 044375 A (NISSAN CHEMICAL INDUSTRIES, LTD.;JAPAN) 26. Februar 1991 (1991-02-26)
- DORN H ET AL: "ALKYL 1-ACYLIERTER PYRAZOLIDONE-(3) UND SYNTHESES 2-SUBSTITUIERTER PYRAZOLIDONE-(3) SOWIE 1-SUBSTITUIERTER 5-HYDROXY-PYRAZOLE" JOURNAL FUER PRAKTISCHE CHEMIE, Bd. 313, Nr. 1, 1. Januar 1971 (1971-01-01), Seiten 115-128, XP000605975 ISSN: 0021-8383 in der Anmeldung erwähnt
- DORN H ET AL: "DIE SYNTHESE VON 3(5)-HYDROXY-PYRAZOL SOWIE VON 5-HYDROXY-1-METHYL- UND 5-HYDROXY-1-CYCLOHEXYL-PYRAZOL" JOURNAL FUER PRAKTISCHE CHEMIE, Bd. 313, Nr. 6, 1. Januar 1971 (1971-01-01), Seiten 1118-1124, XP000605974 ISSN: 0021-8383 in der Anmeldung erwähnt
- DATABASE WPI Section Ch, Week 198640 Derwent Publications Ltd., London, GB; Class E13, AN 1986-261531 XP002130079 -& JP 61 189271 A (UBE IND LTD), 22. August 1986 (1986-08-22) in der Anmeldung erwähnt
- HOUBEN-WEYL: "Methoden der organischen Chemie. Band E8b: "Hetarene III / Teil 2", Seiten 459-464" 1994 , GEORG THIEME VERLAG , STUTTGART DE XP002130086 Seite 462, Absätze 2,3

## Beschreibung

Die vorliegende Erfindung betrifft ein Verfahren zur Herstellung von substituierten Hydroxypyrazolen, sowie neue Zwischenprodukte.

Niedere 1-Alkyl-5-hydroxy-pyrazole sind schon bekannte Verbindungen, die als Zwischenprodukte von Wirkstoffen in Agrochemikalien, insbesondere zur Herstellung von Herbiziden, verwendet werden (siehe WO 97/23135, WO 97/19087, US 5,631,210, WO 97/12885, WO 97/08164, ZA 9510980A, WO 97/01550, WO 96/31507, WO 96/30368, WO 96/25412).

Bisher sind als Verfahren zur Herstellung von niederen 1-Alkyl-5-hydroxy-pyrazolen folgende Synthesen bekannt:
1. eine Darstellung, bei der 2-Methyl-1-(p-toluolsulfonyl)-3-pyrazolidon oder 2-Methyl-1-acetyl-pyrazolidon hydrolysiert wird (J. Prakt. Chem. 1971, 313, 115-128 und J. Prakt. Chem. 1971, 313, 1118-1124).
2. ein Variante, bei der 5-Hydroxy-1-alkylpyrazol-4-carbonsäurealkylester durch Cyclisierung eines Alkoxymethylenmalonsäuredialkylesters mit niederen Alkylhydrazinen aufgebaut wird, anschließend zu diesem Reaktionsprodukt eine wäßrige Mineralsäure-Lösung zugesetzt, und die Hydrolyse und Decarboxylierung gleichzeitig durchgeführt wird (siehe JP 61257974, JP 60051175, JP 58174369, JP 58140073 und JP 58140074 sowie US 4643757).
3. eine Synthese, bei der Propiolsäureethylester mit Methylhydrazin zu 5-Hydroxy-1-methyl-pyrazol umgesetzt wird (Annalen 1965, 686, 134-144).
4. eine Syntheseroute, bei der 3-Hydrazinopropionsäureester, die durch Addition von Hydrazin an Acrylsäureester entstehen, mit Aldehyden zu den entsprechenden Hydrazonen umgesetzt und anschließend cyclisiert werden (siehe JP 06166666, JP 61229852 und JP 61268659 sowie EP 240001).
5. ein Synthesevariante, bei der eine 5-Hydroxy-1-methyl-pyrazol-3-carbonsäure thermisch gespalten wird (Chem. Ber. 1976, 109, S. 261).
6. ein Verfahren, bei dem 3-Alkoxyacrylsäureester mit Methylhydrazin zu 1-Methyl-5-hydroxy-pyrazol umgesetzt werden (siehe JP-A-61/189271).
7. EP-A-0203428 beschreibt die Herstellung von 1-Alkyl- bzw. 1-Alkenyl-5-hydroxypyrazolen durch Umsetzung von Ethoxymethylenmalonsäurediethylestern mit Alkyl- bzw. Alkenylhydrazinen und anschließender Hydrolyse mit Mineralsäure und Decarboxylierung.
8. Aus Houben-Weyl: Methoden der Organischen Chemie, Band8b:"Hetarene III/ Teil 2", Georg Thieme Verlag, 1994, 459-464 ist die Umsetzung von 3-Ethoxyacrylsäureestern mit Hydrazin zu 3(5)-Hydroxypyrazolen bekannt. Außerdem wird dort die Umsetzung von Phenylhydrazin mit 3-Ethoxy-3-chlor-acrylsäurechlorid über die entsprechenden Acrylsäurehydrazide zu 3-Ethoxy-5-hydroxy-1-phenyl- und 5-Ethoxy-3-hydroxy-1-phenyl-1H-pyrazolen beschrieben.

Die oben beschriebenen Synthesen sind in verschiedener Hinsicht nachteilig:

Bei dem oben genannten 1. Syntheseweg ist der Verfahrensprozeß mehrstufig und kompliziert. Das Einfügen und Entfernen einer Schutzgruppe ist umständlich, bedeutet zusätzliche Stufenzahlen und vermindert die Ausbeute.

Die 2. Darstellungsmöglichkeit ist mehrstufig, außerdem entstehen neben den 1-Alkyl-5-hydroxy-pyrazolen gleichzeitig die Regioisomeren 1-Alkyl-3-hydroxy-pyrazole, die aufwendig von den Zielverbindungen abgetrennt werden müssen. Zudem ist die Synthese mit einer schlechten C-Ausbeute verbunden, da ein C4-Baustein eingesetzt wird, wovon am Ende des Verfahrens wieder ein C-Atom abgespalten werden muß.

Bei der 3. Synthesevariante, die lediglich die Darstellung von 1-Methyl-5-hydroxy-pyrazol beschreibt, ist es unabdingbar, weit überstöchiometrische Mengen an Methylhydrazin einzusetzen, wodurch das Verfahren unwirtschaftlich ist. Daneben muß das ebenfalls entstehende Isomer 3-Hydroxy-1-methylpyrazol bei der Reinigung aufwendig von 1-Methyl-5-hydroxy-pyrazol getrennt werden. Des weiteren ist dieses Verfahren aufgrund des hohen Preises von Propiolsäureester unwirtschaftlich.

Bei der 4. Alternative ist der Verfahrensprozeß mehrstufig und kompliziert. Die letzte Stufe des aufwendigen Verfahrens liefert nur schlechte Ausbeuten und eine Vielzahl von Nebenprodukten.

Bei dem 5. Synthesezugang ist zur thermischen Abspaltung eine hohe Temperatur erforderlich, die Ausbeute ist mit 6% sehr gering.

Beim 6. Syntheseweg, der lediglich die Darstellung von 1-Methyl-5-hydroxy-pyrazol beschreibt, werden aufwendig darzustellende und teure 3-Alkoxyacrylsäureester eingesetzt. Die Herstellung von 3-Alkoxyacrylsäureester erfolgt durch Umsetzung von Methanol mit teuren Propiolsäurestern (Tetrahedron Lett. 1983, 24, 5209, J. Org. Chem. 1980, 45, 48, Chem. Ber. 1966, 99, 450, Chem. Lett. 1996, 9, 727-728), durch Umsetzung teurer und aufwendig zu synthetisierenden α,α-Dichlor-diethylether mit Bromessigsäureestern (Zh. Org. Khim. 1986, 22, 738), durch Umsetzung von Bromessigsäureestern mit Trialkylformiaten (Bull. Soc. Chim. France 1983, N 1-2, 41-45) und durch Abspaltung von Methanol aus 3,3-Dialkoxypropionsäureestern (DE 3701113) (erhältlich durch Umsetzung des teuren Propiolsäuremethylester mit Methanol (J. Org. Chem. 1976, 41, 3765), durch Umsetzung von 3-N-Acetyl-N-alkyl-3-methoxy-propionsäureestern mit Methanol (J. Org. Chem. 1985, 50, 4157-4160, JP 60-156643), durch Umsetzung von Acrylsäureestern mit Alkylaminen und Essigsäureanhydrid (J. Org. Chem. 1985, 50, 4157-4160), durch Umsetzung von Keten mit Trialkylorthoformiat (DK 158462), durch Palladium- und gleichzeitiger Kupfer-katalysierte Reaktion von Acrylsäureestern mt Methanol
(DE 4100178.8), durch Umsetzung von Trichloracetylchlorid mit Vinylethylether (Synthesis 1988, 4, 274), durch Umsetzung von α,α,α-Trichlor-ß-methoxy-buten-2-on mit Methanol (Synthesis 1988,4, 274) und durch Umsetzung der Natriumsalze von 3-Hydroxyacrylsäureestern mit Alkoholen (DE 3691605)) eingesetzt werden. Die schlechte Zugänglichkeit der 3-Alkoxyacrylsäureester macht die Synthese nach 6. daher unwirtschaftlich. Nach JP 189 271/86 wird außerdem lediglich die Isolierung des 5-Hydroxy-1-methyl-pyrazols als Hydrochlorid beschrieben, jedoch keine Aussage zur Isolierung und Reinigung der freien Base beschrieben. Versucht man die in JP-A-61/189271 beschriebenen Reaktionsbedingungen anzuwenden und die freie Base zu isolieren, erhält man nur sehr geringe Ausbeuten, die für eine großtechnische Herstellung von Hydroxypyrazolen unwirtschaftlich sind.

Folglich können diese Syntheserouten als wirtschaftliche und effiziente Verfahren zur Herstellung von 1-Alkyl-5-hydroxy-pyrazolen nicht zufriedenstellen. Dies trifft insbesondere zu für den Fall der technischen Herstellung der 1-Alkyl-5-hydroxy-pyrazole in großen Mengen. Aufgabe der vorliegenden Erfindung war es daher, ein alternatives Herstellungsverfahren zur Herstellung von 1-Alkyl-5-hydroxy-pyrazolen zur Verfügung zu stellen, das die oben genannten Nachteile der bisher bekannten Herstellungsmethoden nicht aufweist.

Diese Aufgabe wird überraschend durch das erfindungsgemäße Verfahren gelöst.

Gegenstand der Erfindung ist ein Verfahren zur Herstellung von Verbindungen der Formel I 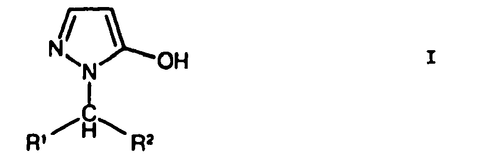 in der R¹ Wasserstoff, eine aliphatische Gruppe mit 1 - 8 Kohlenstoffatomen, C₁-C₆-Alkoxy-carbonyl, C₁-C₆-Alkylthiocarbonyl oder ein cyclisches Ringsystem mit 3-14 Ringatomen bedeutet, und
R² Wasserstoff, eine aliphatische Gruppe mit 1 - 8 Kohlenstoffatomen, oder
R¹ und R² zusammen mit dem Kohlenstoffatom, an das sie gebunden sind, ein cyclisches Ringsystem mit 3 - 14 Ringatomen oder ein bicyclisches Ringsystem mit 7-14 Ring-atomen bedeuten,
umfassend als Ausgangs- oder Zwischenprodukte die Herstellung von Verbindungen der Formel II 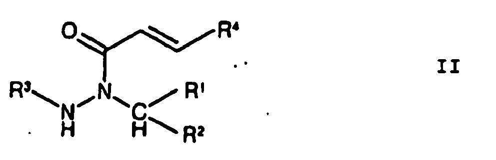 worin R³ eine C₁-C₆-Alkylcarbonylgruppe und R⁴ eine C₁-C₆-Alkoxygruppe bedeutet, und R¹ und R² die oben angegebene Bedeutung besitzen, wobei diese unter geeigneten Reaktionsbedingungen zu Verbindungen der Formel I cyclisiert werden.

Das erfindungsgemäße Verfahren ermöglicht die Herstellung von Verbindungen der Formel I in einer hohen Ausbeute. Die Cyclisierung erfolgt mit Ausbeuten von mindestens 80 %, bei sterisch weniger anspruchsvollen Resten R von mindestens 90 %. Sterisch weniger anspruchsvolle Reste sind insbesondere solche Reste, bei denen die Gruppe -CHR¹R² eine Gruppe mit 1-6 C-Atomen bedeutet. Vorteilhaft ist ferner, daß sich Diacylhydrazine der allgemeinen Formel II unter besonders leichten Bedingungen, wie z.B. kurzen Reaktionszeiten, in die 1-substituierten-5-Hydroxypyrazolen der allgemeinen Formel I überführen lassen. Die Cyclisierung erfolgt bevorzugt säure- oder basenkatalysiert. Vorteilhaft ist ferner, daß die zur Synthese erforderlichen Ausgangsverbindungen leicht verfügbar und kostengünstig sind. Ein weiterer Vorteil besteht darin, daß die Verbindungen der Formel II in hoher Reinheit erhalten werden. Vorteilhaft ist ferner, daß die Hydroxypyrazole der Formel I in freier Form, d.h. im wesentlichen frei von Säureadditionssalzen, zur Verfügung gestellt werden können. Bei den bisher bekannten Synthesen fielen die Hydroxypyrazole fast immer in Form ihrer Säureadditionssalze, wie z.B. Hydrochloride, an, die in einem zusätzlichen Aufarbeitungsschritt erst in die freien Hydroxypyrazole überführt werden mußten. Hingegen fallen die Hydroxypyrazole bei der erfindungsgemäßen Synthese direkt in Form der freien Base an, die im wesentlichen frei sind von Säureadditionssalzen. Ein weiterer Vorteil des erfindungsgemäßen Verfahrens besteht darin, daß die 5-Hydroxypyrazole regioselektiv erhalten werden. Der Anteil an 3-Hydroxypyrazolen ist gering. Bei der erfindungsgemäßen Synthese ist daher insgesamt die Ausbeute an 5-Hydroxypyrazolen im Vergleich zu den bekannten Herstellungsverfahren höher. Aufgrund der in geringeren Mengen anfallenden Nebenprodukte, wie z.B. der 3-Hydroxypyrazole, kann somit ein wirtschaftlicheres Verfahren zur Herstellung der 5-Hydroxypyrazole durchgeführt werden.

Die Diacylhydrazine der Formel II werden beispielsweise hergestellt durch Umsetzung von Verbindungen der Formel III 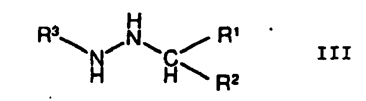 mit aktivierten Alkenylcarbonsäurederivaten der Formel IV 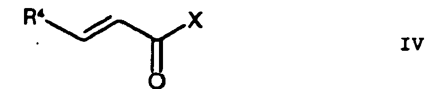 wobei R³ und R⁴ die oben angegebene Bedeutung heben , und X eine geeignete Abgangsgruppe darstellt, unter Abspaltung von HX und anschlieβender Isolierung von Verbindungen der Formel II:

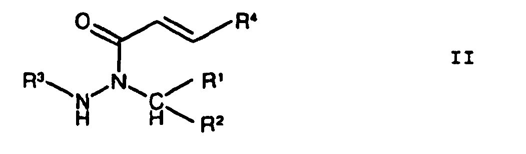

Als Lösungsmittel für die Umsetzung von III mit IV eignet sich vorteilhaft MTBE mit Triethylamin als Base.

Verbindungen der Formel III werden beispielsweise erhalten durch Umsetzung von Verbindungen der Formel V 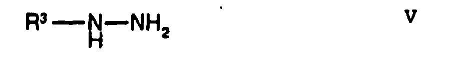 mit Verbindungen der Formel VI 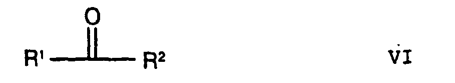 und anschließender katalytischen Hydrierung der intermediär erhaltenen N-Acylhydrazonen der Formel VII 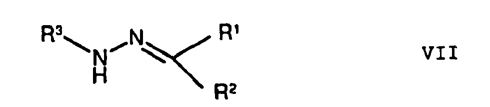 zu den Verbindungen der Formel III. Bei der katalytischen Hydrierung verwendet man als Katalysatoren Raney-Nickel, Pd, Pt oder Adams-Katalysatoren.

Die resultierenden Verbindungen der Formel III können entweder isoliert werden und anschließend mit den aktivierten Alkenylcarbonsäurederivaten der Formel IV umgesetzt werden, oder auch vorteilhaft direkt aus der Reaktionslösung mit den Verbindungen der Formel IV umgesetzt werden. Im letzten Fall kann auf eine Aufreinigung der Zwischenprodukte III verzichtet werden. Die Reaktionsbedingungen für die Hydrierung sind in der Literatur näher beschrieben, z.B. bei Verwendung von H₂/Nickelkatalysatoren: DE 1003215; H₂/Ray-Ni: OD 84-007127/02; mit H₂/PtO₂: Acta. Chem. Scand. 1967, 21, 2363-2366; mit H₂/Pt: Ann. Chimica 1968, 58, 1336-1353; mit H₂/Pd: Indian J. Chem. 1964, 10, 423-424.)

Die Cyclisierung von Verbindungen der Formel II erfolgt säure- oder basenkatalysiert, wobei gleichzeitig die Gruppe R³ am endständigen Stickstoffatom und die Gruppe R⁴ abgespalten werden. Vorzugsweise erfolgt die Cyclisierung unter Verwendung von Mineralsäuren, wie z.B. Salzsäure oder Schwefelsäure.

Das erfindungsgemäße Verfahren zur Herstellung von Verbindungen der Formel I kann durch folgendes Reaktionsschema zusammengefaßt werden, wobei beispielsweise R¹ eine Alkylgruppe, R² Wasserstoff, R³ die Gruppe H₃C-CO- und R⁴ eine Alkoxygruppe bedeuten:

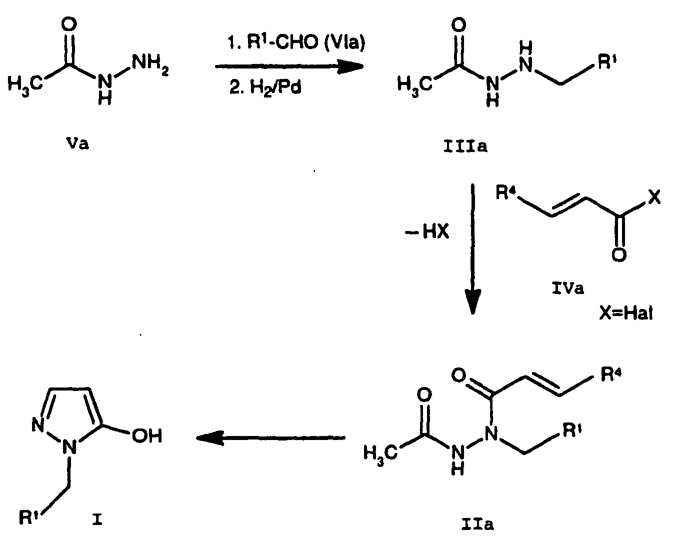

Die Ausbeute bei der Addition von Ketonen oder Aldehyden der Formel VI an Hydrazine der Formel V und die anschließende Hydrierung beträgt jeweils mindestens 80 %, häufig im Bereich zwischen 85 bis 90 %. Die Acylierung von Verbindungen der Formel III zu Verbindungen der Formel II erfolgt in einer Ausbeute von mindestens 80 %, im obigen Fall im Bereich von 80 bis 90 %.

Bei der Definition der verschiedenen Reste in den allgemeinen Formeln stehen die angegebenen Begriffe, entweder für sich allein (wie z.B. "C₁-C₆-Alkyl") oder als Teile bzw. in Kombination mit anderen zusammengesetzten chemischen Gruppen (wie z.B. Halogen-"C₁-C₆-Alkyl", etc.) grundsätzlich als Sammelbegriff für eine Gruppe von Verbindungen. Für den Fall, daß einige der genannten Reste ein- oder mehrfach substituiert sind, so können die Substituenten grundsätzlich gleich oder verschieden sein. Die verwendeten Begriffe bedeuten im einzelnen:
Aliphatische Gruppen: Geradkettiges oder verzweigtes Alkyl, Alkenyl oder Alkinyl mit jeweils bis zu 8 Kohlenstoffatomen, wobei diese Gruppen ein- oder mehrfach substituiert sein können durch Halogen, Halogen-C₁-C₆-alkyl, C₁-C₆-Alkoxy, C₁-C₆-Alkoxycarbonyl, Phenoxy oder Benzyloxy.
Alkyl: Geradkettige oder verzweigte Alkylgruppen mit 1-6, vorzugsweise 1-4 C-Atomen, beispielsweise die folgenden Gruppen: Methyl, Ethyl, Propyl, 1-Methylethyl, Butyl, 1-Methylpropyl, 2-Methylpropyl und 1,1-Dimethylethyl; Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
Alkenyl: Geradkettige oder verzweigte Alkenylgruppen mit 2-6, vorzugsweise 2-4 C-Atomen, beispielsweise die folgenden Gruppen: Ethylen,Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl und 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-l-en-1-yl, 1,2-Dimetyl-but-2-en-1-yl, 1,2-Dimethyl-but-3-en-1-yl, 1,3-Dimetyl-but-1-en-1-yl, 1,3-Dimethyl-but-2-en-1-yl, 1,3-Dimetyl-but-3-en-1-yl, 2,2-Dimethyl-but-3-en-1-yl, 2,3-Dimetyl-but-1-en-1-yl, 2,3-Dimethyl-but-2-en-1-yl, 2,3-Dimetyl-but-3-en-1-yl, 3,3-Dimethyl-but-1-en-1-yl, 3,3-Dimetyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl und 1-Ethyl-2-methyl-prop-2-en-1-yl.
Alkinyl: Geradkettige oder verzweigte Alkinylgruppen mit 2-6, vorzugsweise 2-4 C-Atomen, beispielsweise die folgenden Gruppen: Ethinyl,Prop-1-in-1-yl, Prop-2-in-1-yl, 1-Methylethinyl, Butin-1-yl, Butin-2-yl, Butei-3-yl, 1-Methyl-prop-1-in-1-yl, 2-Methyl-prop-1-in-1-yl, 1-Methyl-prop-2-in-1-yl und 2-Methyl-prop-2-in-1-yl, Pentin-1-yl, Pentin-2-yl, Pentin-3-yl, Pentin-4-yl, 1-Methyl-but-1-in-1-yl, 2-Methyl-but-1-in-1-yl, 3-Methyl-but-1-in-1-yl, 1-Methyl-but-2-in-1-yl.
Alkoxy: Geradkettige oder verzweigte Alkoxygruppen mit 1-6, vorzugsweise 2-4 C-Atomen, beispielsweise Methoxy, Ethoxy, Isopropyloxy, 2-Methyl-but-1-yloxy.
Halogenalkyl: Alkylgruppen wie zuvor genannt, die durch ein bis drei Halogenatome substituiert sind, wie z.B. Trifluormethyl, Difluormethyl, Dichlormethyl, 1,1-Dichlorethyl, 1,2-Dichlorethyl.
Halogen: Fluor, Chlor, Brom oder Jod, vorzugsweise Fluor oder Chlor.
Cyclisches Ringsystem: Ein monocyclisches Ringsystem mit 3-14, vorzugsweise 3- 8 Ringatomen, wobei die Ringatome Kohlenstoff, Stickstoff oder Schwefel sein können. Carbocyclische Ringsysteme besitzen vorzugsweise 3 - 8 C-Atome, wie z.B. Cyclopropyl, Cyclobutyl, Cyclopentyl, Cyclohexyl, Cycloheptyl oder Cyclooctyl. Die cyclischen Ringsysteme können auch eine oder mehrere Doppelbindungen enthalten (C₃-C₈-Cycloalkenylgruppen). Aromatische Ringsysteme sind insbesondere Phenyl und Naphthyl. Für den Fall, daß die cyclischen Ringsysteme heterocyclische Ringsysteme darstellen, können ein, zwei oder drei Kohlenstoffatome durch Heteroatome, wie z.B. O, N, S, ersetzt sein. Grundsätzlich können die cyclischen Ringsysteme aromatisch, teilhydriert oder vollständig hydriert sein. Die cyclischen Ringsysteme können ferner beliebig substituiert sein. Als Substituenten kommen beispielsweise die folgenden Gruppen in Frage: C₁-C₆-Alkyl, Halogen, Halogen-C₁-C₆-alkyl, Amino, Hydroxy, Oxo, C₁-C₆-Alkyl-amino, Di-C₁-C₆-Alkyl-amino, Sulfhydryl, Sulfonyl, C₁-C₆-Alkyl-sulfonyl, etc..
Bicyclisches Ringsystem: Ein bicyclisches Ringsystem mit 7-14 Ringatomen, vorzugsweise 7-10 Ringatomen, wobei die Ringatome Kohlenstoff und ein oder zwei Stickstoffatome sein können. Die Ringsysteme können auch eine oder zwei Doppelbindungen enthalten. Grundsätzlich können die Ringsysteme ein- oder mehrfach substituiert sein durch Alkyl, Alkoxy, Hydroxy, Oxo oder Halogen. Beispielhaft kommen in Frage: Adamantyl, Camphyl, Camphenyl, Norbornyl.

Für den Fall, daß R¹ und R² zusammen mit dem C-Atom, an das sie gebunden sind, ein cyclisches Ringsystem darstellen, versteht man darunter die oben genannten cyclischen Systeme, vorzugsweise C₃-C₈-Cycloalkylgruppen, wie beispielsweise die folgenden: Cyclopropyl, Cyclobutyl, Cyclopentyl, Cyclohexyl, Cycloheptyl oder Cyclooctyl, sowie deren ungesättigten Derivate mit ein-, zwei- oder drei Doppelbindungen im Ringsystem.

Für den Fall, daß R¹ ein cyclisches Ringsystem mit 3-14 Ring-atomen darstellt, versteht man darunter vorzugsweise die folgenden Gruppen: C₃-C₁₄-cycloalkyl, C₃-C₁₄-Cycloalkenyl, aromatische Gruppen, wie Phenyl, Naphthyl, sowie deren teilhydrierten Derivate.

Für den Fall, daß der aliphatische Rest, insbesondere die Alkylgruppe, substituiert ist, kommen bevorzugt folgende Substituenten in Frage: Halogen, C₁-C₆-Alkyl, C₁-C₆-Alkoxycarbonyl. Der aliphatische Rest kann ein- oder mehrfach substituiert sein. In diesem Sinne kommen für R¹ bevorzugt folgenden Bedeutungen in Frage: Difluormethyl, Trifluormethyl, Ethoxycarbonyl, 1,1-Diethoxy-methylen, 1,1-Di-methoxy-methylen.

R² bedeutet vorzugsweise ein Wasserstoffatom.

R³ bedeutet eine unter sauren oder basischen Bedingungen bzw. thermisch leicht abspaltbare Gruppe, Alkylcarbonyl, insbesondere Acetyl, Propionly, Butyryl, bevorzugt Acetyl.

R⁴ bedeutet ebenfalls eine unter sauren oder basischen Bedingungen bzw. thermisch leicht abspaltbare Gruppe, Alkoxy, insbesondere 2-Methyl-prop-1-yloxy (-O-CH₂-CH(CH₃)₂).

Beschrieben werden ferner Verbindungen der Formel I, die im wesentlichen frei sind von Säureadditionssalzen. Ferner Verbindungen der Formel I, wobei R¹ vorzugsweise C₄-C₆-Alkyl, Halogen-C₁-C₆-Alkyl, C₁-C₆-Alkoxy-carbonyl oder C₁-C₆-Alkylthio-carbonyl bedeutet. Diese Verbindungen sind wertvolle Ausgangsstoffe zur Herstellung von weiteren chemischen Substanzgruppen, die einen Hydroxypyrazolrest enthalten, wie z.B. Verbindungen mit herbizider Wirkung, wie bereits eingangs erwähnt.

Das erfindungsgemäße Verfahren eignet sich insbesondere für die Herstellung der Verbindungen der Formel I, wobei die folgenden Reste für sich allein oder jeweils in Kombination miteinander folgende Bedeutungen haben:
1. R¹ und R² bilden zusammen ein bicyclisches Ringsystem, vor allem Adamantyl, Norbornyl, Camphyl.
3. R¹: Wasserstoff; Alkyl; Alkyl, das ein- oder zweifach substituiert ist durch Alkoxy; Phenyl; Halogenalkyl; Alkoxycarbonyl. In diesem Sinne ist R¹ vorzugsweise: Methyl, Ethyl, n-Propyl, i-Propyl; Diethoxymethyl, 2,2-Diethoxy-eth-1-yl; Trifluormethyl; Methoxycarbonyl, Ethoxycarbonyl.
4. R²: Wasserstoff, Alkyl.

Gegenstand der vorliegenden Erfindung sind außerdem neue Verbindungen der Formel II (Diacylhydrazine), die als Zwischenprodukte verwendet werden können. Die Gruppe -CHR¹R² besitzt hierbei die zuvor genannten Bedeutungen. R¹ ist insbesondere Wasserstoff, eine aliphatische Gruppe mit 1-8 Kohlenstoffatomen, C₁-C₆-Alkoxy-carbonyl, C₁-C₆-Alkylthio-carbonyl oder einen cyclisches Ringsystem mit 3-14 Ringatomen. R² ist insbesondere Wasserstoff. R¹ und R² bilden zusammen insbesondere eine Cycloalkylgruppe oder ein bicyclisches Ringsystem in dem oben definierten Sinn.

Die folgende Tabelle zeigt neue Zwischenverbindungen der Formel II, die nach den in den Ausführungsbeispielen beschriebenen Methoden hergestellt werden können:

**Tabelle 1: Verbindungen der Formel II**

| | | | |
|---|---|---|---|
| 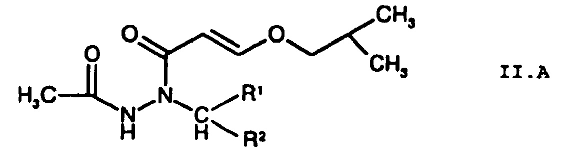 | | | |

| Nr. II . A | R² | R¹ | R¹ und R² |
|---|---|---|---|
| | | | |
| 1. | H | CH₃ | |
| 2. | H | C₂H₅ | |
| 3. | H | n-C₃H₇ | |
| 4. | H | i-C₃H₇ | |
| 5. | H | n-C₄ H₉ | |
| 6. | H | sek-C₄H₉ | |
| 7. | H | tert-C₄H₉ | |
| 8. | H | CH₂-Phenyl | |
| 9. | H | CF₃ | |
| 10. | H | CO-OC₂H₅ | |
| 11. | H | CH(OC₂H₅)₂ | |
| 12. | H | CH₂-CH(OC₂H₅)₂ | |
| 13. | CH₃ | CH₃ | |
| 14. | CH₃ | C₂H₅ | |
| 15. | CH₃ | n-C₃H₇ | |
| 16. | CH₃ | i-C₃H₇ | |
| 17. | CH₃ | n-C₄H₉ | |
| 18. | CH₃ | sek-C₄H₉ | |
| 19. | CH₃ | tert-C₄H₉ | |
| 20. | CH₃ | CH₂-Phenyl | |
| 21. | CH₃ | CF₃ | |
| 22. | CH₃ | CO-OC₂H₅ | |
| 23. | CH₃ | CH (OC₂H₅) ₂ | |
| 24. | CH₃ | CH₂-CH (OC₂H₅)₂ | |
| 25. | - | - | Cyclopropyl |
| 26. | - | - | Cyclobutyl |
| 27. | - | - | Cyclopentyl |
| 28. | - | - | Cyclohexyl |
| 29. | - | - | Cycloheptyl |
| 30. | - | - | Cyclooctyl |
| 31. | - | - | 2-Adamantyl |
| 32. | - | - | 2-Norbornyl |
| 33. | - | - | Camphyl |

Beschrieben sind ferner Verbindungen der Formel III (Hydrazone), die als Zwischenprodukte verwendet werden können. Die Gruppe -CHR¹R²R besitzt hierbei die zuvor genannten Bedeutungen. R¹ ist insbesondere Wasserstoff, eine aliphatische Gruppe mit 1-8 Kohlenstoffatomen, die ein- oder mehrfach durch Halogen oder C₁-C₆-Alkoxy substituiert sein kann, C₁-C₆-Alkoxy-carbonyl, C₁-C₆-Alkylthio-carbonyl oder einen cyclisches Ringsystem mit 3 - 14 Ringatomen. Insbesondere bedeutet R¹ Trifluormethyl, Methoxycarbonyl, Ethoxycarbonyl, Dimethoxy-methyl, Diethoxylmethyl, 2,2-Dimethoxy-ethyl oder 2,2-Diethoxy-ethyl.

Die folgende Tabelle zeigt Zwischenverbindungen der Formel III, die analog zu den in den Ausführungsbeispielen beschriebenen Methoden hergestellt werden können:

**Tabelle 2: Verbindungen der Formel III**

| | | | |
|---|---|---|---|
| 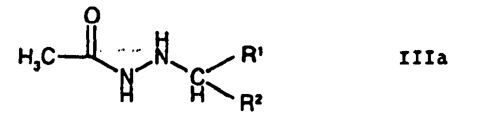 | | | |

| Nr. III.a | R² | R¹ | R¹ und R² |
|---|---|---|---|
| | | | |
| 1. | H | CF₃ | - |
| 2. | H | CO-OC₂H₅ | - |
| 3. | H | CH(OC₂H₅) ₂ | - |
| 4. | H | CH₂-CH(OC₂H₅)₂ | - |
| 5. | CH₃ | CH₃ | - |
| 6. | H | Cyclopentyl | - |
| 7. | - | - | 2-Adamantyl |
| 8. | - | - | 2-Norbornyl |
| 9. | - | - | 2-Camphyl |

Das erfindungsgemäße Verfahren wird anhand der folgenden Ausführungsbeispiele näher erläutert:

### Beispiel 1

Darstellung von 1-Ethyl-5-hydroxypyrazol

### a) N-Acyl-N'-ethylhydrazin

192 g N-Acyl-N'-ethylhydrazon werden in 1.4 1 Methanol mit 40 g Rayney-Nickel in einem Hydrierautoklav vorgelgt. Man hydriert bei 20 bis 110°C und 4 bis 40 bar Wasserstoffdruck, bis die Wasserstoffaufnahme beendet ist. Nach Beendigung der Hydrierung wird vom Katalysator abgetrennt und das Lösungsmittel im Vakuum entfernt. Es werden 190 g (95%) N-Acyl-N'-ethylhydrazin erhalten.

### b) Umsetzung von N-Acyl-N'-ethylhydrazon mit Isobutoxyacrylsäurechlorid

In 950 g tert-Butylmethylether suspendiert man 230 g N-Acyl-N'-ethylhydrazin und 194 g Triethylamin. Man tropft insgesamt 282 g Säurechlorid zu, anschließend versetzt man mit 630 g Wasser und läßt nachrühren. Man trennt die Phasen, trocknet die organische Phase und entfernt das Lösungsmittel. So erhält man 348 g (99%) des unsymmetrischen Diacylhydrazons.

1H-NMR (d6-DMSO): δ = 0.9 (6 H), 1.0 (3 H), 1.9 (3 H), 3.5 82 H), 3.6 (2 H9, 5.6 (1 H), 7.3 (1 H), 10 (1 H).

### c) 1-Ethyl-5-hydroxypyrazol

348 g unsymmetrisches Diacylhydrazin werden in 2000 g 10 proz. Schwefelsäure vorgelegt und auf 90°C erhitzt. Nach 1 h wird auf Raumtemp. gekühlt und mit Natronlauge auf pH 4 gestellt. Die Mischung wird eingeengt und der Rückstand destilliert. Man erhält so 164 g (97%) 1-Ethyl-5-hydroxyparazol.

### Beispiel 2

Darstellung von 1-Benzyl-5-hydroxypyrazol

### a) N-Acyl-N'benzylhylhydrazin

Zu 115.5 g siedendem Essigsäuremethylester tropft man 50 g Hydrazin-Hydrat, anschließend wird 1.5 h bei Rückfluß weitergerührt. Man kühlt auf 17°C, versetzt mit 200 ml Methanol und gibt dann 106 g Benzaldehyd zu. Nachrühren für 35 Min. bei Raumtemp. und nachfolgendes Einengen ergibt 199 g der Iminoverbindung, die ohne weitere Reinigung direkt umgesetzt wird.

66.3 g der oben hergestellten Iminoverbindung werden in Gegenwart von 6 g Raney-Nickel in 120 ml Methanol für 12 h bei 90°C bei einem Druck von 100 bar Wasserstoff im Autoklav hydriert. Der Rückstand wird filtriert, das Lösungsmittel entfernt, wobei man 48.4 g des Hydrierproduktes erhält.

### b) Umsetzung von N-Acyl-N'-benzylhydrazon mit Isobutoxyacrylsäurechlorid

Man legt 37.6 g N-Acyl-N'-benzyl-hydrazin in 190 ml Methylacetat vor und gibt 35.8 g Natriumacetat zu. Bei 11°C 39 g Säurechlorid zu, anschließend versetzt man mit 80 ml Wasser. Man trennt die Phasen und erhält nach Einengen 79.3 g wertprodukt.

### c) 1-Benzyl-5-hydroxypyrazol

79.2 g unsymmetrisches Diacylhydrazin werden in 70g 50 %-iger Schwefelsäure vorgelegt. Man läßt 35 Minuten bei 85-90°C rühren und stellt anschließend mit 136 g 23 %-iger NaOH-Lösung in Methanol auf einen pH von 4.9. Nach Filtration wird die Filtrat eingeengt und der Rückstand aus Essigsäureethylester umkristallisiert. Man erhält so 35 g 1-Benzyl-5-hydroxy-pyrazol.

### Beispiel 3

Analog zu den oben beschriebenen Verfahren wurden folgende Verbindungen hergestellt:

| Bsp.Nr. | Struktur | physikalische- und NMR-Daten |
|---|---|---|
| 3.1 | 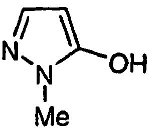 | 1H-NMR (d6-DMSO): 3.5 (s, 3 H), 5.2 (d, 1 H), 7.1 (d, 1 H), 9.5 (br., 1 H). |
| 3.2 | 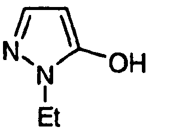 | Fp. 94?C. |
| | | 1H-NMR (d6-DMSO): 1.3 (t, 3 H), 3.9 (q, 2 H), 5.3 (d, 1 H), 7.3 (d, 1 H), 10.4 (br., 1 H). |
| 3.3 | 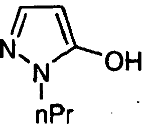 | Kp. (1 mbar): 114?C. |
| | | 1H-NMR (d6-DMSO): 0.8 (t, 3 H), 1.6 (m, 2 H), 3.7 (t, 2 H), 5.3 (d, 1 H), 7.0 (d, 1 H). |
| 3.4 | 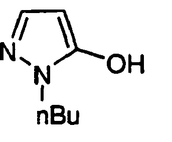 | Kp. (0.5 mbar): 107-108?C. |
| | | 1H-NMR (d6-DMSO): 0.9 (t, 3 H), 1.2 (m, 2 H), 1.7 (m, 2 H), 3.8 (t, 2 H), 5.2 (d, 1 H), 7.0 (d, 1 H), 9.1 (br., 1 H). |
| 3.5 | 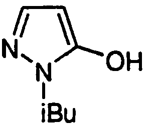 | Kp. (2 mbar): 135?C. |
| | | 1H-NMR (d6-DMSO): 0.9 (d, 6 H), 2.1 (sept., 1 H), 3.5 (d, 2 H), 5.2 (d, 1 H), 7.0 (d, 1 H), 10.6 (br., 1 H). |
| 3.6 | 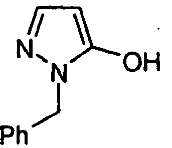 | 1H-NMR (d6-DMSO): 5.1 (s, 2 H), 5.3 (s, 1 H), 7.1-7.3 (m, 6 H), 11.1 (br., 1 H). |
| 3.7 | 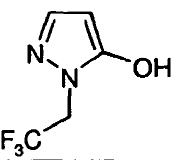 | 1H-NMR (d6-DMSO): 4.7 (q, 2 H), 5.4 (d, 1 H), 7.3 (d, 1 H9, 11.4 (br., 1 H). |
| 3.8 | 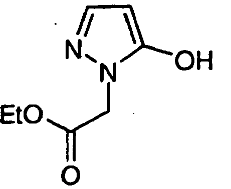 | 1H-NMR (d6-DMSO): 1.2 (t, 2 H), 4.1 (q, 2 H), 4.7 (s, 2 H), 5.3 (d, 1 H), 7.2 (d, 1 H), 11.2 (br., 1 H). |
| 3.9 | 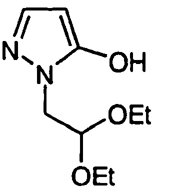 | 1H-NMR (d6-DMSO):1.0 (t, 6 H), 3.3 (m, 2 H), 3.6 (m, 2 H), 3.9 (d, 2 H), 4.7 (t, 1 H), 5.3 (d, 1 H), 7.1 (d, 1 H), 11.0 (br., 1 H). |
| 3.10 | 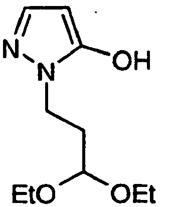 | 1H-NMR (d6-DMSO): 1.1 (t, 6 H), 1.9 (m, 2 H), 3.4 (m, 2 H), 3.6 (m, 2 H), 3.9 (m, 2 H), 4.5 (m, 1 H), 5.3 (d, 1 H), 7.1 (d, 1 H), 11.0 (br., 1 H). |
| 3.11 | 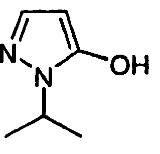 | 1HNMR (d6-DMSO): d = 1.3 (d, 6 H), 4.3 (sept., 1 H), 5.2 (d, 1 H), 7.1 (d, 1 H), 11 (br, 1 H). |

### Beispiel 4

Darstellung von 1-Isopropyl-5-hydroxypyrazol

### a) N-Isopropyl-N'-acetylhydrazid (Verbindung vom Typ III mit R³=H₃CCO; -CHR¹R² = Isopropyl)

56,2 g (0,76 mol) Acetylhydrazid (1), 51,2 g (0,88 mol) Aceton, 200 ml Methanol und 24 g Rayney-Nickel werden 3 h bei Raumtemperatur gerührt, anschließend läßt man 2 h bei 80°C und einem Wasserstoffdruck von 100 bar rühren. Man saugt von vorhandenem Feststoff ab, engt ein und destilliert den Rückstand (0,2 bar, Sdp. 108-113°C). Man erhält so 299 g (85 %) der Titelverbindung.

### b) N-Isopropyl-N-(3-butoxyacrylsäureamid)-N'-acetylhydrazid (Verbindung vom Typ II mit R³=H₃CCO; -CHR¹R² = Isopropyl; R⁴ = -O-CH₂-CH (CH₃)₂)

Man legt 299 g (2,59 mol) der unter a) erhaltenen Verbindung und 425 g Natriumacetat in 1,2 1 Methylacetat vor und tropft 390 g (2,4 mol) 3-Butoxyacrylsäurechlorid zu, läßt 10 Minuten nahrühren um nochmals 31 g (0,19 mol) 3-Butoxyacrylsäurechlorid zuzutropfen. Nach 15 Minuten versetzt man mit 900 ml Wasser und trennt die Phasen. Das Lösungsmittel wird einrotiert und der Rückstand destilliert (0,4 mbar, Übergang 120°C).
Ausbeute: 565 g (90 %) der Titelverbindung.

### c) 1-Isopropyl-5-hydroxypyrazol

Zu 497 g conc. HCl gibt man unter Kühlung 563 g (2,32 mol) der unter b) erhaltenen Verbindung und läßt bei Raumtemperatur rühren. Man stellt mit 50 %iger NaOH auf pH 4.4 und engt ein. Anschließend versetzt man mit Methanol, saugt ab, entfernt vom Filtrat das Lösungsmittel und destilliert den Rückstand (0,3 bar, Sdp. 153°C), danach kristallisiert man aus Ethylacetat um. Schmp. 117-118°C
Ausbeute: 264 g (90 %) der Titelverbindung.
1HNMR (d6-DMSO): δ = 1.3 (d, 6 H), 4.3 (sept., 1 H), 5.2 (d, 1 H), 7.1 (d, 1 H) , 11 (br, 1 H).

## Patentansprüche

1. Verfahren zur Herstellung von Hydroxypyrazolen der Formel I 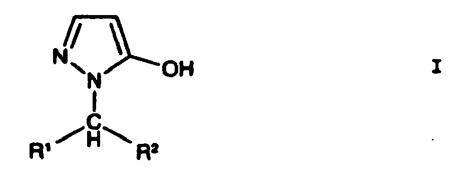 wobei
R¹ Wasserstoff, geradkettiges oder verzweigtes Alkyl, Alkenyl oder Alkinyl mit jeweils 1 bis 8 Kohlenstoffatomen, wobei diese Gruppen ein- oder mehrfach substituiert sein können durch Halogen, Halogen-C₁-C₆-alkyl, C₁-C₆-Alkoxy, C₁-C₆-Alkoxycarbonyl, Phenyloxy oder Benzyloxy, C₁-C₆-Alkoxycarbonyl, C₁-C₆-Alkylthiocarbonyl oder
ein cyclisches Ringsystem mit 3-14 Ringatomen bedeutet, wobei das cyclische Ringsystem ein- oder mehrfach substituiert sein kann durch C₁-C₆-Alkyl, Halogen, Halogen-C₁-C₆-alkyl, Amino, Hydroxy, Oxo, C₁-C₆-Alkylamino, Di-C₁-C₆-alkylamino, Sulfhydryl, Sulfonyl oder C₁-C₆-Alkylsulfonyl, und
R² Wasserstoff, geradkettiges oder verzweigtes Alkyl, Alkenyl oder Alkinyl mit jeweils 1 bis 8 Kohlenstoffatomen bedeutet, wobei diese Gruppen ein- oder mehrfach substituiert sein können durch Halogen, Halogen-C₁-C₆-alkyl, C₁-C₆-Alkoxy, C₁-C₆-Alkoxycarbonyl, Phenyloxy oder Benzyloxy, oder
R¹ und R² zusammen mit dem Kohlenstoffatom, an das sie gebunden sind, ein cyclisches Ringsystem mit 3-14 Ringatomen oder ein bicyclisches Ringsystem mit 7 - 14 Ringatomen bedeutet, wobei das cyclische Ringsystem ein- oder mehrfach substituiert sein kann durch C₁-C₆-Alkyl, Halogen, Halogen-C₁-C₆-alkyl, Amino, Hydroxy, Oxo, C₁-C₆-Alkylamino, Di-C₁-C₆-alkylamino, Sulfhydryl, Sulfonyl oder C₁-C₆-Alkylsulfonyl und das bicyclische Ringsystem ein- oder mehrfach substituiert sein kann durch C₁-C₆-Alkyl, C₁-C₆-Alkoxy, Hydroxy, Oxo oder Halogen,
umfassend die Herstellung einer Verbindung der Formel II 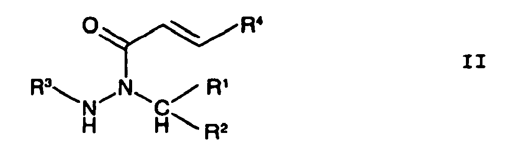 worin R³ eine C₁-C₆-Alkylcarbonylgruppe und R⁴ eine C₁-C₆-Alkoxygruppe bedeutet, und anschließende Cyclisierung der Verbindungen der Formel II zu Verbindungen der Formel I.

2. Verfahren nach Anspruch 1 umfassend die Umsetzung einer Verbindung der Formel III 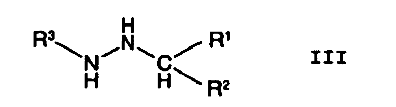 in der R³, R² und R¹ die oben angegebene Bedeutungen haben, mit aktivierten Alkenylcarbonsäurederivaten der Formel IV 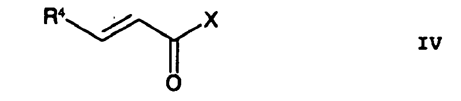 in der R⁴ die oben angegebene Bedeutung hat und X eine geeignete Abgangsgruppe darstellt, zu Verbindungen der Formel II.

3. Verfahren nach Anspruch 1 oder 2 umfassend die Herstellung einer Verbindung der Formel III, **dadurch gekennzeichnet, daß** man eine Verbindung der Formel V 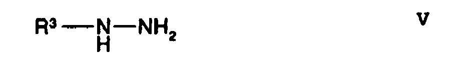 mit einer Verbindung der Formel VI 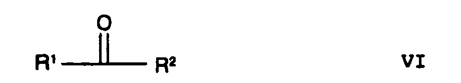 umsetzt und die entstehenden Verbindungen der Formel VII 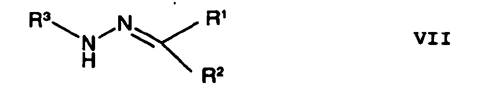 anschließend zu Verbindungen der Formel III hydriert.

4. Verfahren nach Anspruch 1, **dadurch gekennzeichnet, daß** die Cyclisierung von Verbindungen der Formel II zu Verbindungen der Formel I bei Temperaturen zwischen 0° und 100°C erfolgt.

5. Verfahren nach Anspruch 4, wobei als Lösungsmittel Wasser, Alkohole, THF oder Dioxan oder Mischungen davon verwendet werden.

6. Verfahren nach einem der Ansprüche 1, 4 oder 5, wobei die Cyclisierung in Gegenwart einer Mineralsäure oder Lewis-Säure erfolgt.

7. Verfahren nach Anspruch 2, **dadurch gekennzeichnet, daß** die Umsetzung von Verbindungen der Formel III mit Verbindungen der Formel IV bei Temperaturen von -10°C bis 100°C erfolgt.

8. Verfahren nach Anspruch 7, **dadurch gekennzeichnet, daß** die Umsetzung bei Temperaturen zwischen 10° und 30°C erfolgt.

9. Verfahren nach Anspruch 7 oder 8, **dadurch gekennzeichnet, daß** als Lösungsmittel Ether verwendet wird.

10. Verfahren nach Anspruch 7 oder 8, **dadurch gekennzeichnet, daß** als Lösungsmittel THF, MTBE oder 1,2-Dimethoxyethan oder Mischungen davon verwendet werden.

11. Verfahren nach einem der Ansprüche 2 bis 10, **dadurch gekennzeichnet, daß** als HX-Fänger Alkali- oder Erdalkalisalze von Carbonsäuren oder Verbindungen der Formel III verwendet werden.

12. Verfahren nach einem der Ansprüche 1 - 11, wobei die Gruppe -CHR¹R² ein bicyclisches Ringsystem aus der Gruppe Adamantyl, Norbornyl oder Camphyl ist.

13. Verfahren nach einem der Ansprüche 1-11, wobei die Gruppe -CHR¹R² C₃-C₈-Cycloalkyl bedeutet.

14. Verfahren nach einem der Ansprüche 1 - 11, wobei R¹ ein Wasserstoffatom darstellt.

15. Verfahren nach einem der Ansprüche 1 bis 11 oder 14, wobei R² eine C₁-C₆-Alkylgruppe bedeutet.

16. Diacylhydrazine der Formel II 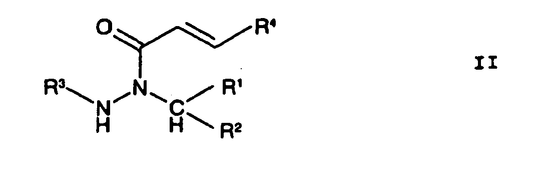 wobei R¹, R², R³ und R⁴ die in Anspruch 1 angegebene Bedeutungen haben.

17. Diacylhydrazine nach Anspruch 16, wobei R³ Acetyl und R⁴ 2-Methyl-1-propyloxy bedeutet.

## Claims

1. A process for preparing hydroxypyrazoles of the
formula I 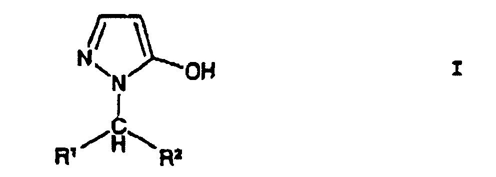 where
R¹ is hydrogen, straight-chain or branched alkyl, alkenyl or alkynyl having in each case from 1 to 8 carbon atoms, where these groups may be substituted by one or more halogen atoms, halo-C₁-C₆-alkyl groups, C₁-C₆-alkoxy, C₁-C₆-alkoxycarbonyl, phenyloxy or benzyloxy, C₁-C₆-alkoxycarbonyl, C₁-C₆alkylthiocarbonyl or a cyclic ring system having 3 - 14 ring atoms, where the cyclic ring system may be monosubstituted or polysubstituted by C₁-C₆-alkyl, halogen, halo-C₁-C₆-alkyl, amino, hydroxy,
oxo, C₁-C₆-alkylamino, di-C₁-C₆-alkylamino, sulfhydryl, sulfonyl or C₁-C₆-alkylsulfonyl, and
R² is hydrogen, straight-chain or branched alkyl, alkenyl or alkynyl having in each case from 1 to 8 carbon atoms, where these groups may be substituted by one or more halogen atoms, halo-C₁-C₆-alkyl groups, C₁-C₆-alkoxy, C₁-C₆-alkoxycarbonyl, phenyloxy or benzyloxy, or
R¹ and R² together with the carbon atom to which they are bound form a cyclic ring system having 3 - 14 ring atoms or a bicyclic ring system having 7 - 14 ring atoms, where the cyclic ring system may be monosubstituted or polysubstituted by C₁-C₆-alkyl, halogen, halo-C₁-C₆-alkyl, amino, hydroxy, oxo, C₁-C₆-alkylamino, di-C₁-C₆-alkylamino, sulfhydryl, sulfonyl or C₁-C₆-alkylsulfonyl, and the bicyclic ring system may be monosubstituted or polysubstituted by C₁-C₆-alkyl, C₁-C₆-alkoxy, hydroxy, oxo or halogen,
comprising the preparation of a compound of the formula II 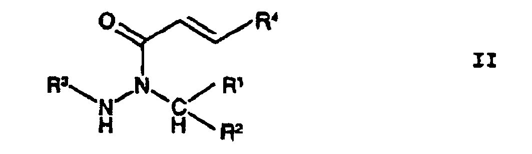 in which R³ is a C₁-C₆-alkylcarbonyl group and R⁴ is a C₁-C₆-alkoxy group, and subsequent cyclization of the compound of the formula II to form a compound of the formula I.

2. The process according to claim 1 comprising the reaction of a compound of the formula III 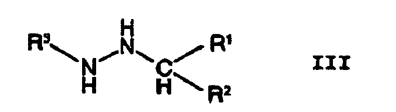 in which R³, R² and R¹ are as defined above, with activated alkenylcarboxylic acid derivatives of the formula IV 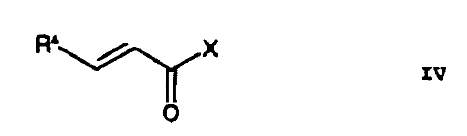 in which R⁴ is as defined above and X is a suitable leaving group, to form a compound of the formula II.

3. The process according to claim 1 or 2 comprising the preparation of a compound of the formula III, wherein a compound of the formula V 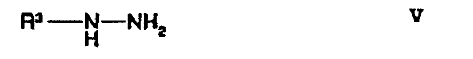 is reacted with a compound of the formula VI 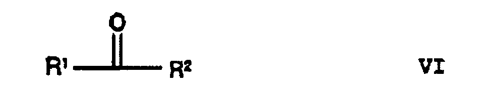 and the resulting compound of the formula VII 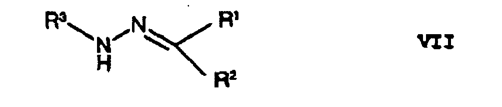 is subsequently hydrogenated to form a compound of the formula III.

4. The process according to claim 1, wherein the cyclization of compounds of the formula II to give compounds of the formula I is carried out at from 0° to 100°C.

5. The process according to claim 4, wherein water, alcohols, THF or dioxane or mixtures thereof are used as solvent.

6. The process according to any of claims 1, 4 and 5, wherein the cyclization is carried out in the presence of a mineral acid or a Lewis acid.

7. The process according to claim 2, wherein the reaction of compounds of the formula III with compounds of the formula IV is carried out at from -10°C to 100°C.

8. The process according to claim 7, wherein the reaction is carried out at from 10° to 30°C.

9. The process according to claim 7 or 8, wherein ether is used as solvent.

10. The process according to claim 7 or 8, wherein THF, MTBE or 1,2-dimethoxyethane or a mixture thereof is used as solvent.

11. The process according to any of claims 2 to 10, wherein alkali metal or alkaline earth metal salts of carboxylic acids or compounds of the formula III are used as HX traps.

12. The process according to any of claims 1 - 11, wherein the group -CHR¹R² is a bicyclic ring system selected from the group consisting of adamantyl, norbornyl and camphyl.

13. The process according to any of claims 1 - 11, wherein the group -CHR¹R² is C₃-C₈-cycloalkyl.

14. The process according to any of claims 1 - 11, wherein R¹ is a hydrogen atom.

15. The process according to any of claims 1 to 11 or 14, wherein R² is a C₁-C₆-alkyl group.

16. A diacylhydrazine of the formula II 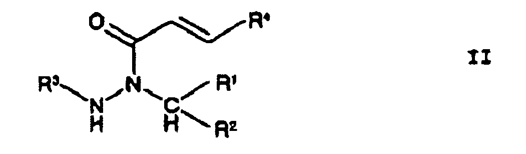 where R¹, R², R³ and R⁴ are as defined in claim 1.

17. A diacylhydrazine according to claim 16, wherein R³ is acetyl and R⁴ is 2-methyl-1-propyloxy.

## Revendications

1. Procédé pour la préparation d'hydroxypyrazoles de formule I 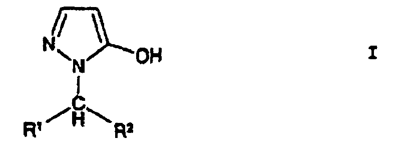 où
R¹ signifie hydrogène, alkyle, alcényle ou alcynyle linéaire ou ramifié comprenant à chaque fois 1 à 8 atomes de carbone, où ces groupes peuvent être monosubstitués ou polysubstitués par halogène, halogéno-C₁-C₆-alkyle, C₁-C₆-alcoxy, C₁-C₆- alcoxycarbonyle, phényloxy ou benzyloxy, C₁-C₆- alcoxycarbonyle, C₁-C₆-alkylthiocarbonyle ou un système cyclique comprenant 3-14 atomes de cycle, où le système cyclique peut être monosubstitué ou polysubstitué par C₁-C₆-alkyle, halogène, halogéno- C₁-C₆-alkyle, amino, hydroxy, oxo, C₁-C₆-alkylamino, di-C₁-C₆-alkylamino, sulfhydryle, sulfonyle ou C₁- C₆-alkylsulfonyle, et
R² signifie hydrogène, alkyle, alcényle ou alcynyle linéaire ou ramifié comprenant à chaque fois 1 à 8 atomes de carbone, où ces groupes peuvent être monosubstitués ou polysubstitués par halogène, halogéno-C₁-C₆-alkyle, C₁-C₆-alcoxy, C₁-C₆- alcoxycarbonyle, phényloxy ou benzyloxy, ou
R¹ et R² ensemble avec l'atome de carbone auquel ils sont liés, signifient un système cyclique comprenant 3-14 atomes de cycle ou un système bicyclique comprenant 7-14 atomes de cycle, où le système cyclique peut être monosubstitué ou polysubstitué par C₁-C₆-alkyle, halogène, halogéno- C₁-C₆-alkyle, amino, hydroxy, oxo, C₁-C₆-alkylamino, di-C₁-C₆-alkylamino, sulfhydryle, sulfonyle ou C₁- C₆-alkylsulfonyle et le système bicyclique peut être monosubstitué ou polysubstitué par C₁-C₆- alkyle, C₁-C₆-alcoxy, hydroxy, oxo ou halogène,
comprenant la préparation d'un composé de formule II 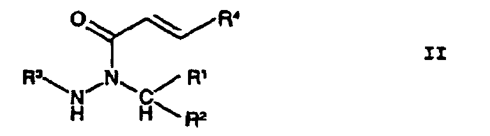 dans laquelle R³ signifie un groupe C₁-C₆-alkylcarbonyle et R⁴ un groupe C₁-C₆-alcoxy, et cyclisation consécutive des composés de formule II en composés de formule I.

2. Procédé selon la revendication 1, comprenant la transformation d'un composé de formule III 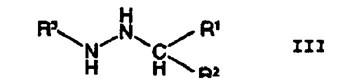 dans laquelle R³, R² et R¹ présentent les significations indiquées ci-dessus, avec des dérivés d'acides alcénylcarboxyliques de formule IV 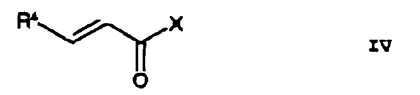 dans laquelle R⁴ a la signification indiquée ci-dessus et X représente un groupe partant approprié, en composés de formule II.

3. Procédé selon la revendication 1 ou 2, comprenant la préparation d'un composé de formule III, **caractérisé en ce qu'**on transforme un composé de formule V 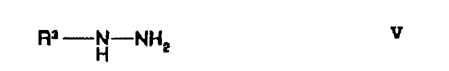 avec un composé de formule VI 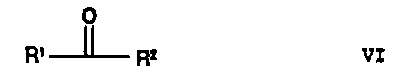 et on hydrogène ensuite les composés formés de formule VII 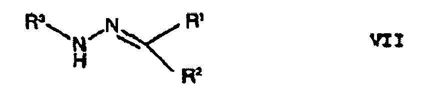 en composés de formule III.

4. Procédé selon la revendication 1, **caractérisé en ce que** la cyclisation de composés de formule II en composés de formule I est réalisée à de températures entre 0°C et 100°C.

5. Procédé selon la revendication 4, où on utilise, comme solvants, de l'eau, des alcools, du THF ou du dioxane ou leurs mélanges.

6. Procédé selon l'une quelconque des revendications 1, 4 ou 5, où la cyclisation est réalisée en présence d'un acide minéral ou d'un acide de Lewis.

7. Procédé selon la revendication 2, **caractérisé en ce que** la transformation de composés de formule III avec des composés de formule IV est réalisée à de températures de -10°C à 100°C.

8. Procédé selon la revendication 7, **caractérisé en ce que** la transformation est réalisée à des températures entre 10°C et 30°C.

9. Procédé selon la revendication 7 ou 8, **caractérisé en ce qu'**on utilise, comme solvant, de l'éther.

10. Procédé selon la revendication 7 ou 8, **caractérisé en ce qu'**on utilise, comme solvants, du THF, du MTBE ou du 1,2-diméthoxyéthane ou leurs mélanges.

11. Procédé selon l'une quelconque des revendications 2 à 10, **caractérisé en ce qu'**on utilise, comme piège de HX, des sels de métal alcalin ou alcalino-terreux d'acides carboxyliques ou des composés de formule III.

12. Procédé selon l'une quelconque des revendications 1-11, où le groupe -CHR¹R² est un système bicyclique du groupe formé par adamantyle, norbornyle ou camphyle.

13. Procédé selon l'une quelconque des revendications 1-11, où le groupe -CHR¹R² signifie C₃-C₈-cycloalkyle.

14. Procédé selon l'une quelconque des revendications 1-11, où R¹ représente un atome d'hydrogène.

15. Procédé selon l'une quelconque des revendications 1 à 11 ou 14, où R² signifie un groupe C₁-C₆-alkyle.

16. Diacylhydrazines de formule II 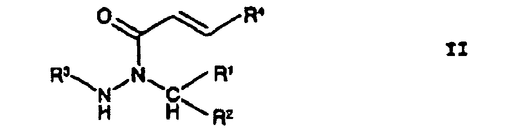 où R¹, R², R³ et R⁴ ont la signification indiquée dans la revendication 1.

17. Diacylhydrazines selon la revendication 16, où R³ signifie acétyle et R⁴ signifie 2-méthyl-1-propyloxy.
